(19) Europäisches Patentamt
European Patent Office
Office européen des brevets

(11) Veröffentlichungsnummer : **0 082 340 B1**

(12) **EUROPÄISCHE PATENTSCHRIFT**

(45) Veröffentlichungstag der Patentschrift : **03.01.90**

(51) Int. Cl.[5] : **C 07 D249/08**, C 07 D233/60, A 01 N 43/64, A 01 N 43/50

(21) Anmeldenummer : **82110875.0**

(22) Anmeldetag : **24.11.82**

(54) **Mikrobizide Triazolyl-ethyl-äther.**

(30) Priorität : **27.11.81 CH 7612/81**

(43) Veröffentlichungstag der Anmeldung : **29.06.83 Patentblatt 83/26**

(45) Bekanntmachung des Hinweises auf die Patenterteilung : **03.01.90 Patentblatt 90/01**

(84) Benannte Vertragsstaaten : **AT BE CH DE FR IT LI LU NL SE**

(56) Entgegenhaltungen :
**EP-A- 0 047 594**
**DE-A- 2 556 319**
**DE-A- 2 654 890**
**DE-A- 2 736 122**
**US-A- 4 123 542**
**Die Akte enthält technische Angaben, die nach dem Eingang der Anmeldung eingereicht wurden und die nicht in dieser Patentschrift enthalten sind.**

(73) Patentinhaber : **CIBA-GEIGY AG**
**Klybeckstrasse 141**
**CH-4002 Basel (CH)**

(72) Erfinder : **Nyfeler, Robert, Dr.**
**Bärenfelserstrasse 8**
**CH-4057 Basel (CH)**
Erfinder : **Meyer, Alfred, Dr.**
**Bristenweg 6**
**CH-4054 Basel (CH)**
Erfinder : **Sturm, Elmar, Dr.**
**Klusstrasse 66**
**CH-4147 Aesch (CH)**

(74) Vertreter : **Zumstein, Fritz jun., Dr. et al**
**Dr. F. Zumstein sen. Dr. E. Assmann Dr. R. Koenigsberger Dipl.-Ing. F. Klingseisen Dr. F. Zumstein jun.**
**Bräuhausstrasse 4**
**D-8000 München 2 (DE)**

**EP 0 082 340 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue, substituierte Triazolyl-ethyl-ether sowie deren Säureadditionssalze, quaternäre Triazoliumsalze und deren Metallkomplexe. Die Erfindung betrifft ferner die Herstellung dieser Substanzen sowie mikrobizide Mittel, die als Wirkstoff mindestens eine dieser Verbindungen enthalten. Die Erfindung betrifft auch die Verwendung der Wirkstoffe oder der Mittel zur Bekämpfung von schädlichen Mikroorganismen.

Es werden hiervon Verbindungen der allgemeinen Formel I

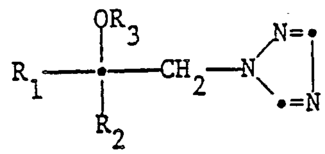

(I)

umfasst,

worin $R_1$ für 2,4-Dichlorphenyl, 2-Chlor-4-fluorphenyl, 2-Fluor-4-chlorphenyl, 2-Chlor-4-bromphenyl, 4-Chlorphenyl oder 4-Fluorphenyl steht, $R_2$ $C_3$-$C_6$-Cycloalkyl oder $C_1$-$C_4$-Alkyl bedeutet und $R_3$ Benzyl, Chlorbenzyl, Dichlorbenzyl, $C_1$-$C_4$-Alkyl oder $C_3$-$C_4$-Alkenyl darstellt; unter Einschluss ihrer Säureadditionssalze, quaternären Triazoliumsalze und deren Metallkomplexe.

Der Ausdruck Triazolyl kennzeichnet IH-1,2,4-Triazolyl. Unter dem Begriff $C_1$-$C_4$-Alkyl sind je nach Zahl der angegebenen Kohlenstoffatome beispielsweise folgende Gruppen zu verstehen: Methyl, Ethyl, Propyl, Butyl, sowie ihre Isomeren, wie z. B. Isopropyl, Isobutyl, tert.-Butyl $C_3$-$C_4$-Alkenyl bedeutet z. B. Propenyl-(1), Allyl, Butenyl-(1), Butenyl-(2) oder Butenyl-(3). $C_3$-$C_6$-Cycloalkyl bedeutet je nach Zahl der Kohlenstoffatome Cyclopropyl, Cyclobutyl, Cyclopentyl, Cyclohexyl.

Die Erfindung betrifft somit die freien organischen Moleküle der allgemeinen Formel I, deren Säureadditionssalze, quaternären Triazoliumsalze und deren Metallkomplexe. Die Freien Moleküle sind bevorzugt.

Beispiele salzbildender Säuren sind anorganische Säuren: Halogenwasserstoffsäure wie Fluorwasserstoffsäure, Chlorwasserstoffsäure, Bromwasserstoffsäure oder Jodwasserstoffsäure sowie Schwefelsäure, Phosphorsäure, phosphorige Säure, Salpetersäure und organische Säuren wie Essigsäure, Trifluoressigsäure, Trichloressigsäure, Propionsäure, Glycolsäure, Thiocyansäure, Milchsäure, Bernsteinsäure, litronensäure, Benzoesäure, Zimtsäure, Oxalsäure, Ameisensäure, Benzolsulfonsäure,. p-Toluolsulfonsäure, Methansulfonsäure, Salicylsäure, p-Aminosalicylsäure, 2-Phenoxybenzoesäure oder 2-Acetoxybenzoesäure.

Metallkomplexe der von Verbindungen des allgemeinen Formel I bestehen aus dem zugrundeliegenden organischen Molekül und einem anorganischen oder organischen Metallsalz, beispielsweise den Halogeniden, Nitraten, Sulfaten, Phosphaten, Acetaten, Trifluoracetaten, Trichloracetaten, Propionaten, Tartraten, Sulfonaten, Salicylaten oder Benzoaten der Elemente der dritten und vierten Hauptgruppe wie Aluminium, Zinn oder Blei sowie der ersten bis achten Nebengruppe wie Chrom, Mangan, Eisen, Zirkonium, Kobalt, Nickel, Kupfer, Zink, Silber oder Quecksilber. Bevorzugt sind die Nebengruppen-Elemente der 4. Periode. Die Metalle können dabei in den verschiedenen ihnen zukommenden Wertigkeiten vorliegen. Die Metallkomplexe der Verbindungen der allgemeinen Formel I können ein- oder mehrkernig auftreten, d. h. sie können ein oder mehrere organische Molekülanteile als Liganden enthalten. Komplexe mit den Metallen Kupfer, Zink, Mangan, Zirkonium und Zinn sind bevorzugt.

Die Verbindungen der allgemeinen Formel I sind bei Raumtemperatur stabile Oele, Harze oder Feststoffe, die sich durch sehr wertvolle mikrobizide Eigenschaften auszeichnen. Sie lassen sich auf dem Agrarsektor oder verwandten Gebieten präventiv und kurativ zur Bekämpfung von pflanzenschädigenden Mikroorganismen einsetzen. Die erfindungsgemässen Wirkstoffe der allgemeinen Formel I zeichnen sich durch eine sehr gute mikrobizide Aktivität und problemlose Anwendung aus.

Unter den Verbindungen der allgemeinen Formel I sind solche von Bedeutung, bei denen $R_3$ $C_1$-$C_4$ Alkyl darstellt.

Zum anderen sind solche Verbindungen der allgemeinen Formel I von besonderer Bedeutung, bei denen $R_2$ Methyl, iso $C_3H_7$ oder sec. $C_4H_9$ ist.

Besonders bevorzugte Einzelsubstanzen sind z. B.:

1-(1H-1,2,4-Triazol-1-yl)-2-benzyloxy-2-(2,4-dichlorphenyl)-pentan,
1-(1H-1,2,4-Triazol-1-yl)-2-methoxy-2-(2,4-dichlorphenyl)-pentan,
1-(1H-1,2,4-Triazol-1-yl)-2-methoxy-2-(4-fluorphenyl)-2-cyclohexylethan,
1-(1H-1,2,4-Triazol-1-yl)-2-n-propoxy-2-(2,4-dichlorphenyl)-pentan,
1-(1H-1,2,4-Triazol-1-yl)-2-n-propoxy-2-(2-chlor-4-fluorphenyl)-pentan,
1-(1H-1,2,4-Triazol-1-yl)-2-methoxy-2-(2,4-dichlorphenyl)-3-methylbutan,
1-(1H-1,2,4-Triazol-1-yl)-2-methoxy-2-(2-chlor-4-fluorphenyl)-pentan und
1-(1H-1,2,4-Triazol-1-yl)-2-(p-chlorbenzyloxy)-2-(2,4-dichlorphenyl)-propan.

Triazolyl-ethan-derivate der allgemeinen Formel I können erfindungsgemäss dadurch hergestellt werden, dass man eine Verbindung der allgemeinen Formel II

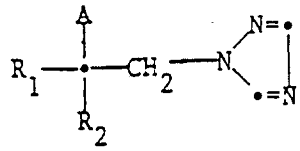

(II)

mit einer Verbindung der allgemeinen Formel III

$$R_3—W \qquad \text{(III)}$$

reagieren lässt, wobei die Substituenten $R_1$, $R_2$ und $R_3$ des allgemeinen wie unter Formel I definiert sind, A und W für —OH, —OM oder eine der üblichen Abgangsgruppen stehen, M ein Alkali- oder Erdalkalimetallatom repräsentiert, mit der Massgabe, dass die Reaktionspartner II und III stets so gewählt werden, dass entweder eine MO- oder HO-Funktion mit einer Abgangsgruppe oder zwei Hydroxyfunktionen miteinander zur Reaktion gelangen.

Unter einer üblichen Abgangsgruppe sollen hier und im folgenden Substituenten wie z. B. Halogene [wie Fluor, Chlor, Brom oder Jod, vorzugsweise Chlor oder Brom]; Sulfonyloxygruppen, bevorzugt $—OSO_2—R_5$; Acyloxygruppen, bevorzugt $—OCO—R_5$ und Isoharnstoffreste bevorzugt

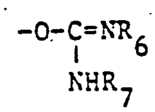

verstanden werden, wobei $R_5$, $R_6$ und $R_7$ unabhängig voneinander für $C_1$-$C_3$-Alkyl, $C_1$-$C_3$-Haloalkyl oder gegebenenfalls durch Halogen, Methyl, Nitro, Trifluormethyl und/oder Methoxy substituiertes Phenyl stehen.

Soweit es sich um Alkohole oder Alkoholate der allgemeinen Formel II handelt (A = OH oder OM), wird man Verbindungen der Allgemeinen Formel I in der Praxis durch übliche Verätherung mit einer Verbindung der allgemeinen Formel III herstellen, worin W ein Halogenid, bevorzugt ein Chlorid oder Bromid, bedeutet. Dabei arbeitet man im Temperaturberich von 0° bis 150 °C entweder ohne Lösungsmittel, bevorzugt aber in atropen Lösungsmitteln wie aromatischen und aliphatischen Kohlenwasserstoffen, Ether und etherartigen Verbindungen (Diethylether, Dioxan, Tetrahydrofuran [THF]), Acetonitril, Dimethylformamid [DMF] und anderen, die dem Fachmann bei Verätherungsreaktionen geläufig sind. Auch die Herstellung im Phasentransfer-Verfahren ist empfehlenswert.

Alkohole der allgemeinen Formel II (A = OH) sind grösstenteils aus der Literatur bekannt oder können analog zu den dort beschriebenen Methoden hergestellt werden. So werden z. B. 1-Phenyl-2-(1H-1,2,4-triazol-1-yl)-ethanol-derivate in der DE-OS-3 042 302, 2-(1H-1,2,4-Triazol-1-yl)-ethanol-derivate in der EP-A-0 015 756 und in der EP-A-0 047 594 und 1-Phenyl-2-(1H-imidazol-1-yl)-ethanol-derivate in der DE-OS-3 042 303 als Fungizide beschrieben. Die erfindungsgemässen Verbindungen der allgemeinen Formel I sind den zitierten Substanzen überlegen.

Die Verbindungen der allgemeinen Formel I besitzen nachbarständig zur Sauerstoffunktion ein Asymmetriezentrum (*)

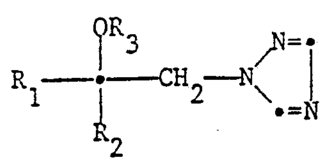

(I)

und können daher in zwei enantiomeren Formen vorliegen. Im allgemeinen entsteht bei der Herstellung dieser Substanzen ein Gemisch beider enantiomerer Formen. Dieses lässt sich durch die üblichen Methoden der Enantiomerentrennung z. B. durch fraktionierte Kristallisation eines diastereomeren Salzgemisches mit einer optisch aktiven starken Säure oder säulenchromatographisch an einem optisch aktiven Träger und mit einem optisch aktiven Eluierungsmittel in die optischen Antipoden aufspalten. Beide Antipoden zeigen eine unterschiedliche mikrobizide Aktivität. Sofern nicht speziell hervorgehoben, liegt bei der Nennung einer Verbindung der allgemeinen Formel I stets ein Gemisch beider enantiomerer Formen vor.

Es hat sich überraschenderweise gezeigt, dass die Aktivsubstanzen der allgemeinen Formel I bzw.

entsprechende Mittel ein für praktische Bedürfnisse sehr günstiges Mikrobizidspektrum vor allem gegen phytopathogene Pilze aufweisen. So besitzen die Verbindungen der allgemeinen Formel I sehr günstige kurative, präventive und systemische Wirkung zum Schutz von Pflanzen, insbesondere Kulturpflanzen, ohne diese nachteilig zu beeinflussen.

Mit den Wirkstoffen der allgemeinen Formel I können an Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wobei auch später zuwachsende Pflanzenteile von derartigen Mikroorganismen verschont bleiben.

Die Wirkstoffe der allgemeinen Formel I sind gegen die den folgenden Klassen angehörenden phytopathogenen Pilze wirksam : Fungi imperfecti (z. B. Botrytis, Helminthosporium, Fusarium Septoria, Cercospora und Alternaria) ; Basidiomyceten (z. B. die Gattungen Hemileia, Rhizoctonia, Puccinia) ; insbesondere wirken sie gegen die Klasse der Ascomyceten (z. B. Venturia, Podosphaera, Erysiphe, Monilinia, Uncinula). Ueberdies wirken die Verbindungen der allgemeinen Formel I systemisch. Sie können ferner als Beizmittel zur Behandlung von Saatgut (Früchte, Knollen, Körner) und Pflanzenstecklingen zum Schutz von Pilzinfektionen sowie gegen im Erdboden auftretende phytopathogene Pilze eingesetzt werden.

Die Erfindung betrifft auch Mittel, die als mindestens eine Aktivsubstanz eine Verbindung der allgemeinen Formel I enthalten, sowie die Verwendung der Mittel oder der Wirkstoffe allein zur Bekämpfung und/oder Verhütung eines Befalls von Mikroorganismen.

Als Zielkulturen für die offenbarten Indikationsgebiete gelten im Rahmen dieser Erfindung beispielsweise folgende Pflanzenarten : Getreide : (Weizen, Gerste, Roggen, Hafer, Reis, Sorghum und Verwandte) ; Rüben : (Zucker- und Futterrüben) ; Kern-, Stein- und Beerenobst : (Aepfel, Birnen, Pflaumen, Pfirsiche, Mandeln, Kirschen, Erd-, Him- und Brombeeren) ; Hülsenfrüchte : (Bohnen, Linsen, Erbsen, Soja) ; Oelkulturen : (Raps, Senf, Mohn, Oliven, Sonnenblumen, Kokos, Rizinus, Kakao, Erdnüsse) ; Gurkengewächse : (Kürbis, Gurken, Melonen) ; Fasergewächse : (Baumwolle, Flachs, Hanf, Jute) ; Citrusfrüchte : (Orangen, Zitronen, Pampelmusen, Mandarinen) ; Gemüsesorten : (Spinat, Kopfsalat, Spargel, Kohlarten, Möhren, Zwiebeln, Tomaten, Kartoffeln, Paprika) ; Lorbeergewächse : (Avocado, Cinnamonum, Kampfer) : oder Pflanzen wie Mais, Tabak, Nüsse, Kaffee, Zuckerrohr, Tee, Weintrauben, Hopfen, Bananen- und Naturkautschukgewächse. Pflanzen sind im Rahmen Erfindung aber auch alle Arten von sonstigen Grünbewachsungen, seien es Zierpflanzen (Compositen), Grasflächen, Böschungen oder allgemeine niedrige Bodenbedeckungen (cover crops).

Wirkstoffe der allgemeinen Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z. B. natürlichen oder regenerierten mineralischen Stoffen, Lösungs-, Dispergier-, Netz-, Haft-, Verdickungs-, Binde- oder Düngemitteln.

Die Verbindungen der allgemeinen Formel I werden dabei in unveränderter Form oder vorzugsweise zusammen mit den in der Formulierungstechnik üblichen Hilfsmitteln eingesetzt und werden daher z. B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z. B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 10 g bis 5 kg Aktivsubstanz (AS) je ha ; bevorzugt 100 g bis 2 kg AS/ha, insbesondere bei 200 g bis 600 g AS/ha.

Die Formulierungen d. h. die den Wirkstoff der allgemeinen Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden). Diese Massnahmen sind dem Fachmann geläufig.

Als Lösungsmittel können in Frage kommen : Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethylether oder -ethylether, Ketone wie Cyclohexanon, stark polare Lösungsmittel wie N-Methyl-2-pyrrolidon, Dimethylsulfoxid oder Dimethylformamid, sowie gegegenenfalls epoxydierte Pflanzenöle wie epoxydiertes Kokosnussöl oder Sojaöl ; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach Art des zu formulierenden Wirkstoffes der allgemeinen Formel I nichtionogene, kation- und/oder anionaktive Tenside mit guten Emulgier-, Dispergier- und Netzeigenschaften in Betracht. Unter Tensiden sind auch Tensidgemische zu verstehen.

Die in der Formulierungstechnik gebräuchlichen Tenside sind u. a. in folgenden Publikationen beschrieben :

« Mc Cutcheon's Detergents and Emulsifiers Annual » BC Publishing Corp., Ringwood New Jersey, 1980
Sisely and Wood, « Encyclopedia of Surface Active Agents », Chemical Publishing Co., Inc. New York, 1980.

Die agrochemischen Zubereitungen enthalten in der Regel 0,1 bis 99 %, insbesondere 0,1 bis 95 % Wirkstoff der allgemeinen Formel I, 99,9 bis 1 %, insbesondere 99,8 bis 5 % eines festen oder flüssigen Zusatzstoffes und 0 bis 25 %, insbesondere 0,1 bis 25 % eines Tensides.

Während als Handelsware eher konzentrierte Mittel bevorzugt werden, verwendet der Endverbraucher in der Regel verdünnte Mittel.

Die nachfolgenden Beispiele dienen zur näheren Erläuterung der Erfindung, ohne dieselbe einzuschränken. Temperaturen sind in Celsiusgraden angegeben. Prozente und Teile beziehen sich auf das Gewicht. Darüberhinaus werden folgende Symbole verwendet : h = Stunde ; d = Tag ; min = Minute ; RT = Raumtemperatur ; N = Normalität ; abs = absolut, wasserfrei.

## Herstellungsbeispiel

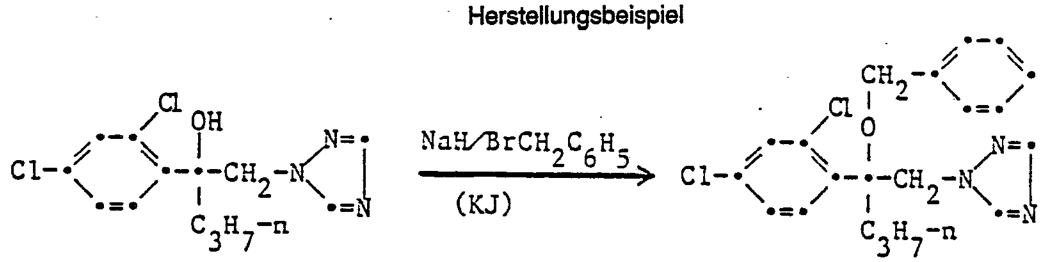


(2)

1-(1H-1,2,4-Triazol-1-yl)-2-benzyloxy-2-(2,4-dichlorphenyl)-pentan

Zu einer Suspension von 2,4 g 55 %igen Natriumhydrid in 100 ml absolutem DMF (anhaftendes Mineralöl wird zuvor mit Hexan weggewaschen) lässt man unter Stickstoffatmosphäre eine Lösung von 15 g 1-(1H-1,2,4-Triazol-1-yl)-2-(2,4-dichlorphenyl)-pentan-2-ol in 40 ml absolutem DMF und 10 ml absolutem THF zutropfen. Nach Abklingen der exothermen Reaktion wird noch 1 h bei 50° weitergerührt, und anschliessend 1 g Kaliumjodid zugegeben. Dann lässt man 9,6 g Benzylbromid in 10 ml DMF zutropfen, rührt 12 h bei RT und giesst das Reaktionsgemisch auf Eiswasser. Die trübe Emulsion wird mit Natriumchlorid gesättigt und mehrfach mit Ethylacetat extrahiert. Die vereinigten Extrakte werden mit halbgesättigter Natriumchlorid-Lösung gewaschen, über Natriumsulfat getrocknet, filtriert und eingeengt. Der Rückstand wird säulenchromatographisch (Silicagel/Dichlormethan) gereinigt. Die erste Fraktion ergibt 15 g eines gelblichen Oels.

Analyse :
Berechnet : C 61,55% H 5,45% N 10,77% Cl 18,17%
Gefunden : C 61,6 % H 5,7 % N 10,5 % Cl 18,0 %.

Die folgende Tabelle enthält die vorgenannte Verbindung sowie weitere, die ähnlich hergestellt wurden.

(Siehe Tabelle 1 Seite 6 f.)

Tabelle 1

Verbindungen der allgemeinen Formel I

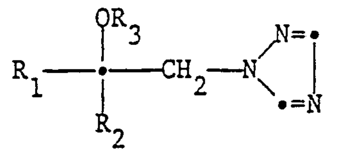

(I)

| Verb. Nr. | $R_1$ | $R_2$ | $R_3$ | Physik. Konst. [°C] |
|---|---|---|---|---|
| 1 | $C_6H_4Cl(4)$ | Cyclohexyl | $CH_3$ | Fp. 118-119 |
| 2 | $C_6H_3Cl_2(2,4)$ | $C_3H_7$-n | $CH_2$-$C_6H_5$ | Oel |
| 3 | $C_6H_4Cl(4)$ | Cyclohexyl | $CH_2$-$C_6H_5$ | Fp. 116-118 |
| 4 | $C_6H_3Cl_2(2,4)$ | $C_3H_7$-n | $CH_3$ | Kp. 170-175/ 0,04 mbar |
| 5 | $C_6H_4F(4)$ | Cyclohexyl | $CH_3$ | Fp. 100-101 |
| 6 | $C_6H_4F(4)$ | Cyclohexyl | $CH_2$-$C_6H_5$ | Fp. 74-75 |
| 7 | $C_6H_3Cl_2(2,4)$ | Cyclohexyl | $C_2H_5$ | |
| 8 | $C_6H_3Cl_2(2,4)$ | $C_3H_7$-n | $CH_2CH = CH_2$ | Oel |
| 9 | $C_6H_3Cl_2(2,4)$ | $C_3H_7$-i | $CH_3$ | Kp. 158-162/ 0,03 mbar |
| 10 | $C_6H_3Cl_2(2,4)$ | $CH_3$ | $CH_2$-$CH = CH_2$ | Fp. 68-70 |
| 11 | $C_6H_3Cl_2(2,4)$ | $CH_3$ | $CH_2$-$C_6H_4Cl(4)$ | |
| 12 | $C_6H_3Cl(2)Br(4)$ | $CH_3$ | $CH_3$ | Oel |
| 13 | $C_6H_4Cl(4)$ | $C_3H_7$-n | $CH_3$ | Kp. 245-250/ 0,05 mbar |
| 14 | $C_6H_3Cl_2(2,4)$ | $C_4H_9$-n | $CH_3$ | Kp. 240-250/ 0,04 mbar |
| 15 | $C_6H_3Cl_2(2,4)$ | $C_4H_9$-n | $CH_2$-$C_6H_5$ | |
| 16 | $C_6H_3Cl_2(2,4)$ | $C_4H_9$-n | $CH_2$-$C_6H_4Cl(4)$ | Oel |
| 17 | $C_6H_4Cl(4)$ | $C_4H_9$-n | $CH_3$ | Kp. 185/195/ 0,01 mbar |
| 18 | $C_6H_4Cl(4)$ | $C_4H_9$-n | $CH_2$-$C_6H_5$ | Oel |
| 19 | $C_6H_3Cl_2(2,4)$ | $CH_3$ | $CH_3$ | Oel |
| 20 | $C_6H_3Cl_2(2,4)$ | $CH_3$ | $CH_2$-$C_6H_5$ | Oel |
| 21 | $C_6H_3Cl_2(2,4)$ | $CH_3$ | $CH_2$-$C_6H_4Cl(4)$ | Oel |
| 22 | $C_6H_3Cl_2(2,4)$ | $CH_3$ | $C_4H_9$-n | Oel |
| 23 | $C_6H_4Cl(4)$ | Cyclopentyl | $CH_3$ | |
| 24 | $C_6H_3Cl_2(2,4)$ | Cyclopentyl | $CH_3$ | Oel |
| 25 | $C_6H_3Cl_2(2,4)$ | Cyclopentyl | $CH_2$-$C_6H_5$ | |
| 26 | $C_6H_3Cl_2(2,4)$ | Cyclopentyl | $CH_2$-$C_6H_3Cl_2(2,4)$ | |
| 27 | $C_6H_3Cl_2(2,4)$ | Cyclopentyl | $C_4H_9$-n | |
| 28 | $C_6H_4F(4)$ | Cyclobutyl | $CH_3$ | Oel |
| 29 | $C_6H_4Cl(4)$ | $C_3H_7$-n | $CH_2C_6H_5$ | Kp. 170-176/ 0,02 mbar |
| 30 | $C_6H_3Cl_2(2,4)$ | Cyclohexyl | $CH_3$ | Kp. 200-210°/ 0,1 mbar |
| 31 | $C_6H_3Cl(2)F(4)$ | $CH_3$ | $CH_2C_6H_5$ | Kp. 250/0,04 mbar |
| 32 | $C_6H_3Cl(2)Br(4)$ | $CH_3$ | $CH_2C_6H_5$ | Kp. 219/0,02 mbar |
| 33 | $C_6H_3Cl_2(2,4)$ | $CH_3$ | $C_3H_7$-n | Fp. 62-64 |
| 34 | $C_6H_3Cl_2(2,4)$ | $CH_3$ | $CH_2$-C = $CH_2$<br>$CH_3$ | Kp. 165-175°/ 0,02 mbar |
| 35 | $C_6H_3Cl_2(2,4)$ | $CH_3$ | $CH_2CH = CHCH_3$ | Kp. 190-200°/ 0,02 mbar |
| 36 | $C_6H_3Cl_2(2,4)$ | $C_3H_7$-n | $CH_2$-C = $CH_2$<br>$CH_3$ | Kp. 170-180°/ 0,02 mbar |
| 37 | $C_6H_3Cl_2(2,4)$ | $C_3H_7$-n | $CH_2CH = CHCH_3$ | Kp. 180-190°/ 0,02 mbar |
| 38 | $C_6H_3Cl_2(2,4)$ | $C_3H_7$-n | $C_3H_7$-n | Oel |
| 39 | $C_6H_3Cl_2(2,4)$ | $C_3H_7$-n | $C_4H_9$-n | Kp. 189-195°/ 0,02 mbar |
| 40 | $C_6H_3Cl(2)F(4)$ | $C_3H_7$-n | $CH_2$-$CH = CH_2$ | Kp. 210/0,04 mbar |
| 41 | $C_6H_3Cl(2)F(4)$ | $C_3H_7$-n | $C_3H_7$-n | Kp. 208/0,04 mbar |

Tabelle 1 (Fortsetzung)
Verbindungen der Zillgemeinen Formel I

| Verb. Nr. | $R_1$ | $R_2$ | $R_3$ | Physik. Konst. [°C] |
|---|---|---|---|---|
| 42 | $C_6H_3Cl(2)F(4)$ | $C_3H_7$-n | $CH_3$ | Kp. 190/0,04 mbar |
| 43 | $C_6H_3Cl(2)F(4)$ | $C_2H_5$ | $CH_3$ | Kp. 148-153/0,02 mbar |
| 44 | $C_6H_3Cl(2)F(4)$ | $C_2H_5$ | $CH_2$-$CH=CH_2$ | Kp. 180-186/0,03 mbar |
| 45 | $C_6H_3Cl_2(2,4)$ | $C_2H_5$ | $CH_3$ | Kp. 170-175/0,04 mbar |
| 46 | $C_6H_3Cl_2(2,4)$ | $C_2H_5$ | $C_3H_7$-n | Kp. 192-198/0,03 mbar |
| 47 | $C_6H_4F(4)$ | $C_2H_5$ | $C_4H_9$-n | Oel |
| 48 | $C_6H_4F(4)$ | $C_2H_5$ | $-CH_2CH=CHCH_3$ | Oel |
| 49 | $C_6H_4F(4)$ | $C_3H_7$-n | $C_3H_7$-n | Oel |
| 50 | $C_6H_4F(4)$ | $CH_3$ | $CH_2$-$C_6H_4Cl(4)$ | Oel |
| 51 | $C_6H_4F(4)$ | $C_2H_5$ | $CH_3$ | viskoses Öl |

Formulierungsbeispiele für Wirkstoffe der allgemeinen Formel I (% = Gewichtsprozent).

| Emulsions-Konzentrate /Spritzpulver | a) | b) | c) |
|---|---|---|---|
| Wirkstoff aus Tabelle 1 | 25% | 40% | 50% |
| Ca-Dodecylbenzolsulfonat | 5% | 8% | 6% |
| Ricinusöl-polyethylenglykolether (36 Mol Ethylenoxid) | 5% | - | - |
| Tributylphenoyl-polyethylenglykolether (30 Mol Ethylenoxid) | - | 12% | 4% |
| Cyclohexanon | - | 15% | 20% |
| Xylolgemisch | 65% | 25% | 20% |

Aus solchen Konzentraten können durch Verdünnen mit Wasser Emulsionen jeder gewünschten Konzentration hergestellt werden. Spritzpulver liegen vor, wenn der Xylol-Anteil durch Kieselsäure und/oder Kaolin ersetzt wird.

| Lösungen | a) | b) | c) | d) |
|---|---|---|---|---|
| Wirkstoff aus Tabelle 1 | 80% | 10% | 5% | 95% |
| Ethylenglykol-monomethyl-ether | 20% | - | - | - |
| Polyethylenglykol M G 400 | - | 70% | - | - |
| N-Methyl-2-pyrrolidon | - | 20% | - | - |
| Epoxydiertes Kokosnussöl | - | - | 1% | 5% |
| Benzin (Siedegrenzen 160-190°C) | - | - | 94% | - |

(MG = Molekulargewicht)
Die Lösungen sind zur Anwendung in Form kleinster Tropfen geeignet.

| Granulate | a) | b) |
|---|---|---|
| Wirkstoff aus Tabelle 1 | 5% | 10% |
| Kaolin | 94% | - |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschliessend im Vakuum abgedampft.

| Stäubemittel | a) | b) |
|---|---|---|
| Wirkstoff aus der Tabelle I | 2% | 5% |
| Hochdisperse Kieselsäure | 1% | 5% |
| Talkum | 97% | - |
| Kaolin | - | 90% |

Durch inniges Vermischen der Trägerstoffe mit dem Wirkstoff erhält man gebrauchsfertige Stäubemittel.

## Biologische Beispiele

### Beispiel 2.1

### Wirkung gegen Puccinia graminis auf Weizen

a) Residual-protektive Wirkung

Weizenpflanzen wurden 6 Tage nach der Aussaat mit einer aus Spritzpulver des Wirkstoffes hergestellten Spritzbrühe (0,002 % Aktivsubstanz) besprüht. Nach 24 Stunden wurden die behandelten Pflanzen mit einer Ureidosporensuspension des Pilzes infiziert. Nach einer Inkubation während 48 Stunden bei 95-100 % relativer Luftfeuchtigkeit und ca. 20 °C wurden die infizierten Pflanzen in einem Gewächshaus bei ca. 22 °C aufgestellt. Die Beurteilung der Rostpustelnentwicklung erfolgte 12 Tage nach der Infektion.

b) Systemische Wirkung

Zu Weizenpflanzen wurden 5 Tage nach Aussaat eine aus Spritzpulver des Wirkstoffes hergestellte Spritzbrühe gegossen (0,0006 % Aktivsubstanz bezogen auf das Bodenvolumen). Nach 48 Stunden wurden die behandelten Pflanzen mit einer Uredosporensuspension des Pilzes infiziert. Nach einer Inkubation während 48 Stunden bei 95-100 % relativer Luftfeuchtigkeit und ca. 20 °C wurden die infizierten Pflanzen in einem Gewächshaus bei ca. 22 °C aufgestellt. Die Beurteilung der Rostpustelnentwicklung erfolgte 12 Tage nach der Infektion.

Verbindungen aus der Tabelle I zeigten gegen Puccinia-Pilze eine gute Wirkung. Unbehandelte aber infizierte Kontrollpflanzen zeigten einen Puccinia-Befall von 100 %. Unter anderen hemmten die Verbindungen Nr. 1-6, 8-14, 17, 19-22, 28, 29, 31-42, 49, 51 den Puccinia-Befall auf 0 bis 5 %.

### Beispiel 2.2

### Wirkung gegen Cercospora arachidicola auf Erdnusspflanzen

Residual-protektive Wirkung

10-15 cm hohe Erdnusspflanzen wurden mit einer aus Spritzpulver der Wirksubstanz hergestellten Spritzbrühe (0,006 % Aktivsubstanz) besprüht und 48 Stunden später mit einer Konidiensuspension des Pilzes infiziert. Die infizierten Pflanzen wurden während 72 Stunden bei ca. 21 °C und hoher Luftfeuchtigkeit inkubiert und anschliessend bis zum Auftreten der typischen Blattflecken in einem Gewächshaus aufgestellt.

Die Beurteilung der fungiziden Wirkung erfolgt 12 Tage nach der Infektion basierend auf Anzahl und Grösse der auftretenden Flecken.

Im Vergleich zu unbehandelten, aber infizierten Kontrollpflanzen (Anzahl und Grösse der Flecken = 100 %), zeigten Erdnusspflanzen, die mit Wirkstoffen aus der Tabelle I behandelt wurden, einen stark reduzierten Cercospora-Befall. So verhinderten die Verbindungen Nr. 1, 2, 4-6, 8-10, 12-14, 17-22, 32-51 in obigem Versuch das Auftreten von Flecken fast vollständig (0 bis 10 %).

### Beispiel 2.3

### Wirkung gegen Erysiphe graminis auf Gerste

a) Residual-protektive Wirkung

Ca. 8 cm hohe Gerstenpflanzen wurden mit einer aus Spritzpulver des Wirkstoffes hergestellten Spritzbrühe (0,002 % Aktivsubstanz) besprüht. Nach 3-4 Stunden wurden die behandelten Pflanzen mit Konidien des Pilzes bestäubt. Die infizierten Gerstenpflanzen wurden in einem Gewächshaus bei ca. 22 °C aufgestellt und der Pilzbefall nach 10 Tagen beurteilt.

b) Systemische Wirkung

Zu ca. 8 cm hohen Gerstenpflanzen wurde eine aus Spritzpulver des Wirkstoffes hergestellte

Spritzbrühe gegossen (0,0006 % Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit Konidien des Pilzes bestäubt. Die infizierten Gerstenpflanzen wurden in einem Gewächshaus bei ca. 22 °C aufgestellt und der Pilzbefall nach 10 Tagen beurteilt.

Verbindungen der allgemeinen Formel I zeigten gute Wirkung gegen Erysiphe-Pilze. Unbehandelte, aber infizierte Kontrollpflanzen zeigten einen Erysiphe-Befall von 100 %. Unter anderen Verbindungen aus der Tabelle I hemmten Verbindungen Nr. 1-14, 16-22, 24, 28, 29, 31-51 den Pilzbefall auf Gerste auf 0 bis 5 %. Besonders wirksam (kein Befall) waren die Verbindungen Nr. 1, 2, 4-6, 7-10, 12-14, 17, 19-21, 29, 31-42 und 49.

Beispiel 2.4

Residual-protektive Wirkung gegen Venturia inaequalis auf Apfeltrieben

Apfelstecklinge mit 10-20 cm langen Frischtrieben wurden mit einer aus Spritzpulver des Wirkstoffes hergestellten Spritzbrühe (0,006 % Aktivsubstanz) besprüht. Nach 24 Stunden wurden die behandelten Pflanzen mit einer Konidiensuspension des Pilzes infiziert. Die Pflanzen wurden dann während 5 Tagen bei 90-100 % relativer Luftfeuchtigkeit inkubiert und während 10 weiteren Tagen in einem Gawächshaus bei 20-24 °C aufgestellt. Der Schorfbefall wurde 15 Tage nach der Infektion beurteilt. Kontrollpflanzen waren 100 %ig befallen. Verbindungen Nr. 1, 2, 4, 5, 12-14, 17, 19-21, 28, 29, 32-42 und 49 hemmten den Krankheitsbefall auf weniger als 10 %. Bei Behandlung mit den Wirkstoffen Nr. 2, 4, 5, 12, 14, 37-42 trat überhaupt kein Befall auf.

Beispiel 2.5

Wirkung gegen Botrytis cinerea auf Aepfeln

Residual protektive Wirkung

Künstlich verletzte Aepfel wurden behandelt, indem eine aus Spritzpulver der Wirksubstanz hergestellte Spritzbrühe (0,02 % Aktivsubstanz) auf die Verletzungsstellen aufgetropft wurde. Die behandelten Früchte wurden anschliessend mit einer Sporensuspension von Botrytis cinerea inokuliert und während einer Woche bei hoher Luftfeuchtigkeit und ca. 20 °C inkubiert.

Das Vorhandensein und die Grösse der Fäulnisstellen an der Frucht dienten zur Bewertung der fungiziden Aktivität. Bei Behandlung mit den Verbindungen Nr. 2, 4, 6, 8, 10, 12, 14, 17, 19, 21 und 28 wurden keine oder fast keine Fäulnisstellen (0-5 % Befall) beobachtet.

**Patentansprüche** (für die benannten Vertragsstaaten : BE, CH, DE, FR, IT, LI, LU, NL, SE)

1. Verbindungen der allgemeinen Formel I, ihre Säureadditionssalze, quaternären Triazoliumsalze und deren Metallkomplexe

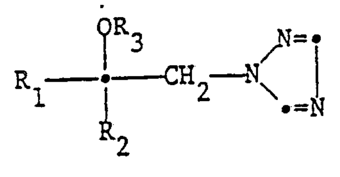

(I)

worin $R_1$ für 2,4-Dichlorphenyl, 2-Chlor-4-fluorphenyl, 2-Fluor-4-chlorphenyl, 2-Chlor-4-bromphenyl, 4-Chlorphenyl oder 4-Fluorphenyl steht, $R_2$ $C_3$-$C_6$-Cycloalkyl oder $C_1$-$C_4$-Alkyl bedeutet und $R_3$ Benzyl, Chlorbenzyl, Dichlorbenzyl, $C_1$-$C_4$-Alkyl oder $C_3$-$C_4$-Alkenyl darstellt.

2. Verbindungen der allgemeinen Formel I nach Anspruch 1, bei der $R_3$ $C_1$-$C_4$-Alkyl darstellt.

3. Verbindungen der allgemeinen Formel I nach Anspruch 1, bei der $R_2$ Methyl, Isopropyl oder sec.-Butyl ist.

4. 1-(1H-1,2,4-Triazol-1-yl)-2-methoxy-2-(2,4-dichlorphenyl)-butan.

5. 1-(1H-1,2,4-Triazol-1-yl)-2-n-propoxy-2-(2,4-dichlorphenyl)-butan.

6. 1-(1H-1,2,4-Triazol-1-yl)-2-methoxy-2-(2,4-dichlorphenyl)-pentan.

7. 1-(1H-1,2,4-Triazol-1-yl)-2-methoxy-2-(2-chlor-4-fluorphenyl)-pentan.

8. Verfahren zur Herstellung der in Anspruch 1 definierten Verbindungen der allgemeinen Formel I, dadurch gekennzeichnet, dass man eine Verbindung der allgemeinen Formel II

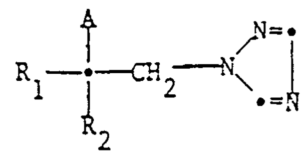

(II)

mit einer Verbindung der allgemeinen Formel III

$R_3—W$

(III)

reagieren lässt, wobei die Substituenten $R_1$, $R_2$ und $R_3$ wie unter der allgemeinen Formel I definiert sind, A und W für —OH, —OM oder eine der üblichen Abgangsgruppen stehen, M ein Alkali- oder Erdalkalimetallatom repräsentiert, mit der Massgabe, dass die Reaktionspartner II und III stets so gewählt werden, dass entweder eine MO- oder HO-Funktion mit einer Abgangsgruppe oder zwei Hydroxyfunktionen miteinander zur Reaktion gebracht werden.

9. Verfahren nach Anspruch 8, dadurch gekennzeichnet, dass A oder W als Abgangsgruppe für ein Halogen, $—OSO_2R_5$, $—OCO—R_5$ oder

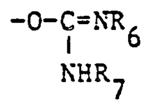

stehen, wobei $R_5$, $R_6$ und $R_7$ unabhängig voneinander $C_1$-$C_3$-Alkyl, $C_1$-$C_3$-Haloalkyl oder gegebenenfalls durch Halogen, Methyl, Nitro, Trifluormethyl und/oder Methoxy substituiertes Phenyl bedeuten.

10. Mittel zur Bekämpfung oder präventiven Verhütung von Mikro-organismen-Befall, dadurch gekennzeichnet, dass es als aktive Komponente mindestens eine Verbindung der allgemeinen Formel I gemäss Anspruch 1 enthält.

11. Mittel gemäss Anspruch 10, dadurch gekennzeichnet, dass es eine Verbindung der allgemeinen Formel I gemäss Anspruch 2 enthält.

12. Mittel gemäss Anspruch 10, dadurch gekennzeichnet, dass es eine Verbindung der allgemeinen Formel I gemäss Anspruch 3 enthält.

13. Mittel gemäss Anspruch 10, dadurch gekennzeichnet, dass es als aktive Komponente 1-(1H-1,2,4-Triazol-1-yl)-2-methoxy-2-(2,4-dichlorphenyl)-butan enthält.

14. Mittel gemäss Anspruch 10, dadurch gekennzeichnet, dass es als aktive Komponente 1-(1H-1,2,4-Triazol-1-yl)-2-n-propoxy-2-(2,4-dichlorphenyl)-butan enthält.

15. Mittel gemäss Anspruch 10, dadurch gekennzeichnet, dass es als aktive Komponente 1-(1H-1,2,4-Triazol-1-yl)-2-methoxy-2-(2,4-dichlorphenyl)-pentan enthält.

16. Mittel gemäss Anspruch 10, dadurch gekennzeichnet, dass es als aktive Komponente 1-(1H-1,2,4-Triazol-1-yl)-2-methoxy-2-(2-chlor-4-fluorphenyl)-pentan enthält.

17. Verfahren zur Bekämpfung oder Verhütung eines Befalls von Pflanzen durch phytopathogene Mikroorganismen, dadurch gekennzeichnet, dass man eine Verbindung der allgemeinen Formel I gemäss einem der Ansprüche 1 bis 7 auf die Pflanze oder deren Standort appliziert.

**Patentansprüche** (für den Vertragsstaat AT)

1. Verfahren zur Herstellung einer Verbindung der allgemeinen Formel I

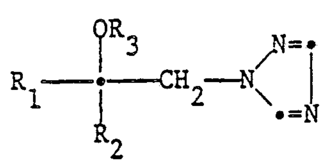

(I)

worin $R_1$ für 2,4-Dichlorphenyl, 2-Chlor-4-fluorphenyl, 2-Fluor-4-chlorphenyl, 2-Chlor-4-bromphenyl, 4-Chlorphenyl oder 4-Fluorphenyl steht, $R_2$ $C_3$-$C_6$-Cycloalkyl oder $C_1$-$C_4$-Alkyl bedeutet und $R_3$ Benzyl, Chlorbenzyl, Dichlorbenzyl, $C_1$-$C_4$-Alkyl oder $C_3$-$C_4$-Alkenyl darstellt, dadurch gekennzeichnet, dass man eine Verbindung der allgemeinen Formel II

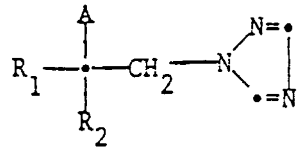

(II)

mit einer Verbindung der allgemeinen Formel III

$$R_3—W \quad (III)$$

reagieren lässt, wobei die Substituenten $R_1$, $R_2$ und $R_3$ wie unter der allgemeinen Formel I definiert sind, A und W für —OH, —OM oder eine der üblichen Abgangsgruppen stehen, M ein Alkali- oder Erdalkalimetallatom repräsentiert, mit der Massgabe, dass die Reaktionspartner II und III stets so gewählt werden, dass entweder eine MO- oder HO-Funktion mit einer Abgangsgruppe oder zwei Hydroxyfunktionen miteinander zur Reaktion gebracht werden.

2. Verfahren nach Anspruch 1, dadurch gekennzeichnet, dass A oder W als Abgangsgruppe für ein Halogen, für $—OSO_2R_5$, $—OCO—R_5$ oder

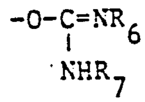

stehen, wobei $R_5$, $R_6$ und $R_7$ unabhängig voneinander $C_1$-$C_3$-Alkyl, $C_1$-$C_3$-Haloalkyl oder gegebenenfalls durch Halogen, Methyl, Nitro, Trifluormethyl und/oder Methoxy substituiertes Phenyl bedeuten.

3. Mittel zur Bekämpfung oder präventiven Verhütung von Mikro-organismen-Befall, dadurch gekennzeichnet, dass es als aktive Komponente mindestens eine Verbindung der allgemeinen Formel I gemäss Anspruch 1 enthält.

4. Mittel gemäss Anspruch 3, dadurch gekennzeichnet, dass es eine Verbindung der allgemeinen Formel I enthält, worin $R_3$ $C_1$-$C_4$-Alkyl darstellt.

5. Mittel gemäss Anspruch 3, dadurch gekennzeichnet, dass es eine Verbindung der allgemeinen Formel I enthält, bei der $R_2$ Methyl, Isopropyl oder sec.-Butyl ist.

6. Mittel gemäss Anspruch 3, dadurch gekennzeichnet, dass es als aktive Komponente 1-51H-1,2,4-Triazol-1-yl)-2-methoxy-2-(2,4-dichlorphenyl)-butan enthält.

7. Mittel gemäss Anspruch 3, dadurch gekennzeichnet, dass es als aktive Komponente 1-(1H-1,2,4-Triazol-1-yl)-2-n-propoxy-2-(2,4-dichlorphenyl)-butan enthält.

8. Mittel gemäss Anspruch 3, dadurch gekennzeichnet, dass es als aktive Komponente 1-(1H-1,2,4-Triazol-1-yl)-2-methoxy-2-(2,4-dichlorphenyl)-pentan enthält.

9. Mittel gemäss Anspruch 3, dadurch gekennzeichnet, dass es als aktive Komponente 1-(1H-1,2,4-Triazol-1-yl)-2-methoxy-2-(2-chlor-4-fluorphenyl)-pentan enthält.

10. Verfahren zur Bekämpfung oder Verhütung eines Befalls von Pflanzen durch phytopathogene Midroorganismen, dadurch gekennzeichnet, dass man eine Verbindung der allgemeinen Formel I gemäss einem der Ansprüche 3 bis 9 auf die Pflanze oder deren Standort appliziert.

**Claims** (for the Contracting States : BE, CH, DE, FR, IT, LI, LU, NL, SE)

1. A compound of the general formula I

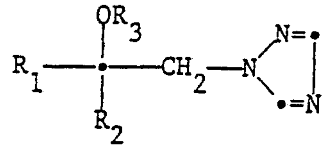

(I),

wherein $R_1$ is 2,4-dichlorophenyl, 2-chloro-4-fluorophenyl, 2-fluoro-4-chlorophenyl, 2-chloro-4-bromophenyl, 4-chlorophenyl or 4-fluorophenyl, $R_2$ is $C_3$-$C_6$cycloalkyl or $C_1$-$C_4$alkyl, and $R_3$ is benzyl, chlorobenzyl, dichlorobenzyl, $C_1$-$C_4$alkyl or $C_3$-$C_4$alkenyl, or an acid addition salt, quaternary triazolium salt or a metal complex thereof.

2. A compound of the general formula I according to claim 1, wherein $R_3$ is $C_1$-$C_4$alkyl.

3. A compound of the general formula I according to claim 1, wherein $R_2$ is methyl, isopropyl or sec-

butyl.

4. 1-(1H-1,2,4-Triazol-1-yl)-2-(2,4-dichlorophenyl) butane.

5. 1-(1H-1,2,4-Triazolyl-1-yl)-2-n-propoxy-2-(2,4-dichlorophenyl) butane.

6. 1-(1H-1,2,4-Triazol-1-yl)-2-methoxy-2-(2,4-dichlorophenyl) pentane.

7. 1-(1H-1,2,4-Triazol-1-yl)-2-methoxy-2-(2-chloro-4-fluorophenyl) pentane.

8. A process for the preparation of a compound of the general formula I as defined in claim 1, which comprises reacting a compound of the general formula II

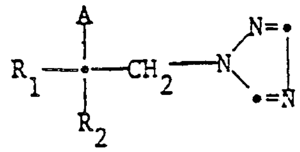

(II)

with a compound of the general formula III

$$R_3—W \qquad (III)$$

in which formulae above the substituents $R_1$, $R_2$ and $R_3$ are as defined for the general formula I, A and W are —OH, —OM or one of the customary leaving groups, M is an alkali metal atom or an alkaline earth metal atom, with the proviso that the reactants II and III are always chosen such that either one MO or HO function is reacted with one leaving group or two hydroxy functions.

9. A process according to claim 8, wherein a leaving group A or W is a halogen atom,

$-OSO_2R_5$, $-OCO-R_5$ or 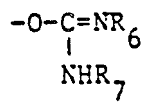

in which $R_5$, $R_6$ and $R_7$ are each independently of one another $C_1$-$C_3$alkyl, $C_1$-$C_3$haloalkyl, phenyl or phenyl which is substituted by halogen, methyl, nitro, trifluoromethyl and/or methoxy.

10. A composition for controlling microorganisms or for preventing infestation by microorganisms, which composition contains as active component at least one compound of the general formula I according to claim 1.

11. A composition according to claim 10, which contains a compound of the general formula I according to claim 2.

12. A composition according to claim 10, which contains a compound of the general formula I according to claim 3.

13. A composition according to claim 10, which contains as active component 1-(1H1,2,4-triazol-1-yl)-2-methoxy-2-(2,4-dichlorophenyl) butane.

14. A composition according to claim 10, which contains as active component 1-(1H-1,2,4-triazolyl-1-yl)-2-n-propoxy-2-(2,4-dichlorophenyl) butane.

15. A composition according to claim 10, which contains active component 1-(1H-1,2,4-triazol-1-yl)-2-methoxy-2-(2,4-dichlorophenyl) pentane.

16. A composition according to claim 10, which contains as active component 1-(1H-1,2,4-triazol-1-yl)-2-methoxy-2-(2-chloro-4-fluorophenyl) pentane.

17. A method of controlling phytopathogenic microorganisms or of preventing infestation of plants by said microorganisms, which comprises applying to the plants or the locus thereof a compound of the general formula I according to any one of claims 1 to 7.

**Claims** (for the Contracting State AT)

1. A process for the preparation of a compound of the general formula I

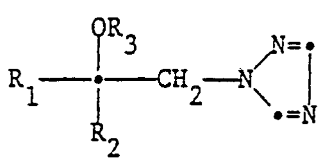

(I)

wherein $R_1$ is 2,4-dichlorophenyl, 2-chloro-4-fluorophenyl, 2-fluoro-4-chlorophenyl, 2-chloro-4-bromophenyl, 4-chlorophenyl or 4-fluorophenyl, $R_2$ is $C_3$-$C_6$cycloalkyl or $C_1$-$C_4$alkyl, and $R_3$ is benzyl, chlorobenzyl, dichlorobenzyl, $C_1$-$C_4$alkyl or $C_3$-$C_4$alkenyl, which process comprises reacting a compound of the general formula II

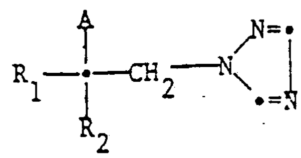

(II)

with a compound of the general formula III

$$R_3—W \qquad (III)$$

in which formulae above the substituents $R_1$, $R_2$ and $R_3$ are as defined for the general formula I, A and W are —OH, —OM or one of the customary leaving groups, M is an alkali metal atom or an alkaline earth metal atom, with the proviso that the reactants II and III are always chosen such that either one MO or HO functions is reacted with one leaving group or two hydroxy functions.

2. A process according to claim 1, wherein a leaving group A or W is a halogen atom, $—OSO_2R_5$, $—OCO—R_5$ or

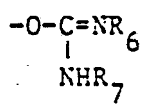

in which $R_5$, $R_6$ and $R_7$ are each independently of one another $C_1$-$C_3$alkyl, $C_1$-$C_3$haloalkyl, phenyl or phenyl which is substituted by halogen, methyl, nitro, trifluoromethyl and/or methoxy.

3. A composition for controlling microorganisms or for preventing infestation by microorganisms, which composition contains as active component at least one compound of the general formula I according to claim 1.

4. A composition according to claim 3, which contains a compound of the general formula I according to claim 1, wherein $R_3$ is $C_1$-$C_4$alkyl.

5. A composition according to claim 3, which contains a compound of the general formula I according to claim 1, wherein $R_2$ is methyl, isopropyl or sec-butyl.

6. A composition according to claim 3, which contains as active component 1-(1H-1,2,4-triazol-1-yl)-2-methoxy-2-(2,4-dichlorophenyl) butane.

7. A composition according to claim 3, which contains as active component 1-(1H-1,2,4-triazolyl-1-yl)-2-n-propoxy-2-(2,4-dichlorophenyl) butane.

8. A composition according to claim 3, contains as active component 1-(1H-1,2,4-triazol-1-yl)-2-methoxy-2-(2,4-dichlorophenyl) pentane.

9. A composition according to claim 3, which contains as active component 1-(1H-1,2,4-triazol-1-yl)-2-methoxy-2-(2-chloro-4-fluorophenyl) pentane.

10. A method of controlling phytopathogenic microorganisms or of preventing infestation of plants by sait microorganisms, which comprises applying to the plants or the locus thereof a compound of the general formula I according to any one of claims 3 to 9.

**Revendications** (pour les Etats contractants : BE, CH, DE, FR, IT, LI, LU, NL, SE)

1. Composés de formule générale I, leurs sels d'addition avec des acides, leurs sels de triazolium quaternaires et leurs complexes métalliques ;

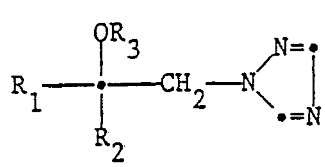

(I)

où $R_1$ représente le 2,4-dichlorophényle, le 2-chloro-4-fluorophényle, le 2-fluoro-4-chlorophényle, le 2-chloro-4-bromophényle, le 4-chlorophényle ou le 4-fluorophényle, $R_2$ représente un cycloalkyle en $C_3$-$C_6$ ou un alkyle en $C_1$-$C_4$ et $R_3$ représente le benzyle, le chlorobenzyle, le dichlorobenzyle, un alkyle en $C_1$-$C_4$ ou un alcényle en $C_3$-$C_4$.

2. Composés de formule générale I selon la revendication 1, dans lesquels $R_3$ représente un alkyle en $C_1$-$C_4$.

3. Composés de formule générale I selon la revendication 1, dans lesquels $R_2$ est le méthyle, l'isopropyle ou le sec.-butyle.

4. 1-(1H-1,2,4-triazol-1-yl)-2-méthoxy-2-(2,4-dichlorophényl)-butane.

5. 1-(1H-1,2,4-triazol-1-yl)-2-n-propoxy-2-(2,4-dichlorophényl)-butane.

6. 1-(1H-1,2,4-triazol-1-yl)-2-méthoxy-2-(2,4-dichlorophényl)-pentane.

7. 1-(1H-1,2,4-triazol-1-yl)-2-méthoxy-2-(2,4-chloro-4-fluorophényl)-pentane.

8. Procédé de préparation des composés de formule générale I définis dans la revendication 1, caractérisé en ce que l'on fait réagir un composé de formule générale II

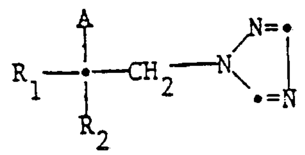

(II)

avec un composé de formule générale III

$R_3—W$ (III)

où les substituants $R_1$, $R_2$ et $R_3$ sont définis comme sous la formule générale I, A et W représentent —OH, —OM ou un des groupes partants usuels, M représente un atome de métal alcalin ou alcalino-terreux, sous réserve que les partenaires réactionnels II et III soient toujours choisis de telle façon qu'une fonction —OM ou —OH soit mise à réagir ensemble avec un groupe partant ou deux fonctions hydroxy.

9. Procédé selon la revendication 8, caractérisé en ce que A ou W représente comme groupe partant un halogène, $—OSO_2R_5$, $—OCO—R_5$ ou

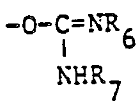

où $R_5$, $R_6$ et $R_7$ représentent, indépendamment l'un de l'autre, un alkyle en $C_1$-$C_3$, un haloalkyle en $C_1$-$C_3$ ou un groupe phényle éventuellement substitué par un halogène, un méthyle, un nitro, un trifluorométhyle et/ou un méthoxy.

10. Agent pour lutter contre ou prévenir l'infestation par les microorganismes, caractérisé en ce qu'il contient, à titre de composant actif, au moins un composé de formule générale I selon la revendication 1.

11. Agent selon la revendication 10, caractérisé en ce qu'il contient un composé de formule générale I selon la revendication 2.

12. Agent selon la revendication 10, caractérisé en ce qu'il contient un composé de formule générale I selon la revendication 3.

13. Agent selon la revendication 10, caractérisé en ce qu'il contient, comme composant actif, le 1-(1H-1,2,4-triazol-1-yl)-2-méthoxy-2-(2,4-dichlorophényl)-butane.

14. Agent selon la revendication 10, caractérisé en ce qu'il contient, comme composant actif, le 1-(1H-1,2,4-triazol-1-yl)-2-n-propoxy-2-(2,4-dichlorophényl)-butane.

15. Agent selon la revendication 10, caractérisé en ce qu'il contient, comme composant actif, le 1-(1H-1,2,4-triazol-1-yl)-2-méthoxy-2-(2,4-dichlorophényl)-pentane.

16. Agent selon la revendication 10, caractérisé en ce qu'il contient, comme composant actif, le 1-(1H-1,2,4-triazol-1-yl)-2-méthoxy-2-(2-chloro-4-fluorophényl)-pentane.

17. Procédé pour lutter contre ou prévenir une infestation des plantes par des microorganismes phytopathogènes, caractérisé en ce que l'on applique un composé de formule générale I selon l'une des revendications 1 à 7 sur la plante ou à son emplacement.

**Revendications** (pour l'Etat contractant AT)

1. Procédé de préparation d'un composé de formule générale I

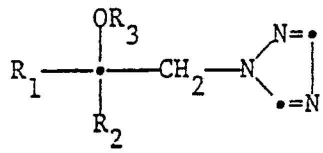

(I)

où $R_1$ représente le 2,4-dichlorophényle, le 2-chloro-4-fluorophényle, le 2-fluoro-4-chlorophényle, le 2-chloro-4-bromophényle, le 4-chlorophényle ou la 4-fluorophényle, $R_2$ représente un cycloalkyle en $C_3$-$C_6$ ou un alkyle en $C_1$-$C_4$ et $R_3$ représente le benzyle, le chlorobenzyle, le dichlorobenzyle, un alkyle en $C_1$-$C_4$ ou un alcényle en $C_3$-$C_4$, caractérisé en ce que l'on fait réagir un composé de formule générale II

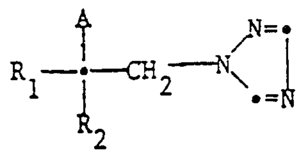

(II)

avec un composé de formule générale III

$$R_3—W \qquad (III)$$

où les substituants $R_1$, $R_2$ et $R_3$ sont définis comme sous la formule générale I, A et W représentent —OH, —OM ou un des groupes partants usuels, M représente un atome de métal alcalin ou alcalino-terreux, sous réserve que les partenaires réactionnels II et III soient toujours choisis de telle façon qu'une fonction —OM ou —OH soit mise à réagir ensemble avec un groupe partant ou deux fonctions hydroxy.

2. Procédé selon la revendication 1, caractérisé en ce que A ou W représente comme groupe partant un halogène, $—OSO_2R_5$, $—OCO—R_5$ ou

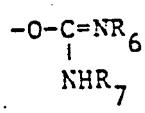

où $R_5$, $R_6$ et $R_7$ représentent, indépendamment l'un de l'autre, un alkyle en $C_1$-$C_3$, un haloalkyle en $C_1$-$C_3$ ou un groupe phényle éventuellement substitué par un halogène, un méthyle, un nitro, un trifluorométhyle et/ou un méthoxy.

3. Agent pour lutter contre ou prévenir l'infestation par les microorganismes, caractérisé en ce qu'il contient, à titre de composant actif, au moins un composé de formule générale I selon la revendication 1.

4. Agent selon la revendication 3, caractérisé en ce qu'il contient un composé de formule générale I où $R_3$ représente un alkyle en $C_1$-$C_4$.

5. Agent selon la revendication 3, caractérisé en ce qu'il contient un composé de formule générale I où $R_2$ est le méthyle, l'isopropyle ou le sec.-butyle.

6. Agent selon la revendication 3, caractérisé en ce qu'il contient, comme composant actif, le 1-(1H-1,2,4-triazol-1-yl)-2-méthoxy-2-(2,4-dichlorophényl)-butane.

7. Agent selon la revendication 3, caractérisé en ce qu'il contient, comme composant actif, le 1-(1H-1,2,4-triazol-1-yl)-2-n-propoxy-2-(2,4-dichlorophényl)-butane.

8. Agent selon la revendication 3, caractérisé en ce qu'il contient, comme composant actif, le 1-(1H-1,2,4-triazol-1-yl)-2-méthoxy-2-(2,4-dichlorophényl)-pentane.

9. Agent selon la revendication 3, caractérisé en ce qu'il contient, comme composant actif le 1-(1H-1,2,4-triazol-1-yl)-2-méthoxy-2-(2-chloro-4-fluorophényl)-pentane.

10. Procédé pour lutter contre ou prévenir une infestation des plantes par des microorganismes phytopathogènes, caractérisé en ce que l'on applique un composé de formule générale I selon l'une des revendications 3 à 9 sur la plante ou à son emplacement.